# EUROPEAN PATENT APPLICATION

(11) **EP 3 053 557 A2**
(43) Date of publication of application: **10.08.2016**
(21) Application number: 14819474.9
(22) Date of filing: 25.09.2014
(51) Int. Cl.: A61F 13/15, A61F 13/472

(54) **ABSORBENT ARTICLE**

(30) Priority: 30.09.2013 JP 2013204166
(71) Applicant: Unicharm Corporation, Ehime 799-0111 (JP)
(72) Inventor: TANIGUCHI, Kenta, Kanonji-shi Kagawa 769-1602 (JP); KITAGAWA, Masashi, Kanonji-shi Kagawa 769-1602 (JP); HARADA, Hiroyuki, Kanonji-shi Kagawa 769-1602 (JP)
(74) Representative: Peter, Julian
(86) International application number: PCT/JP2014/075534
(87) International publication number: WO 2015/002331

(57) **Abstract**

An absorbent article (1) has a top sheet (10), a back sheet (20), an absorber (30), and a pair of side sheets (41) that cover the side edges of the absorber in a width direction. Lateral inner edges (41W) of the side sheets are interposed between the absorber and the top sheet in the thickness direction. The pair of side sheets extend outwardly from the top sheet and the absorber in the width direction. The side sheets have a higher density than the top sheet.

## Description

### Technical Field

The present invention relates to an absorbent article such as a sanitary napkin.

### Background Art

Patent Literature 1 discloses an absorbent article having: a top member covering a top surface and sides of an absorber; and side sheets (barrier members) extending outwardly from the top member and the absorber in a width direction. The top member is formed of a liquid-permeable nonwoven fabric and the side sheets are formed of a nonwoven fabric having a hydrophobic surface.

The absorber of the Patent Literature 1 is directly covered by the top member on the top surface and the sides so that menses will be absorbed not only from a skin-contacting surface side but also from the sides of the absorber. Further, the side sheets extend outwardly from the absorber in the width direction so that menses on the side sheets will be absorbed from the sides of the absorber.

### Citation List

### Patent Literature

Patent Literature 1: JP H7-184957 A

### Summary

Unfortunately, the absorbent article mentioned above has the following problem.

Because the side sheets are formed of a hydrophobic nonwoven fabric, menses tend to remain on the side sheets rather than on the top sheet formed of a hydrophilic nonwoven fabric. Further, menses on the side sheets can cause leakage and soil wearer's clothing. Furthermore, wearer's anxiety about leakage can be increased at the sight of a large amount of menses on the side sheets.

In recent years, absorbent articles have become more popular in developing countries and newly industrialized countries. According to the inventors' research on the usage of absorbent articles in developing countries, especially in those countries where women have just started to use such absorbent articles, some wearers feel uncomfortable carrying absorbent articles with them. Other wearers, even though they do not feel uncomfortable carrying absorbent articles with them, can have difficulties changing and disposing of absorbent articles away from home when needed due to shortage of suitable facilities. Such wearers, compared to wearers who carry absorbent articles with them and change as needed, tend to wear one without change for relatively longer hours during the daytime. As a result of the inventors' research, in newly industrialized countries, absorbent articles were changed minimum every 6 to 8 hours on average.

When an absorbent article is worn for many hours without change during the daytime, it gets typically compressed inwardly in width direction because it is repeatedly pressed between wearer's legs by way of wearer's walking, for example. Being repeatedly compressed inwardly in width direction, the absorbent article becomes contracted in the width direction, and in turn side sheets will receive an increased amount of menses thereon. As the amount of menses received on the side sheets increases, soiling of the wearers' clothing typically increases, and thus wearers' concern about leakage can further increase.

The present invention addresses the above problem with an object of providing an absorbent article that can prevent leakage of menstrual flow in the crotch region of the absorbent article even during long hours of use, thereby easing wearer concerns about leakage during use.

To solve the above problem, there is provided an absorbent article absorbent article (1) according to the present invention comprising: a longitudinal direction (longitudinal direction L); a width direction orthogonal to the longitudinal direction (width direction W) ; a thickness direction; a liquid-permeable top sheet (a top sheet 10); a liquid-impermeable back sheet (a back sheet 20); an absorber (an absorber 30) interposed between the top sheet and the back sheet; and a pair of side sheets (side sheets 41) covering side edges of the absorber in the width direction, wherein inner edges of the pair of side sheets (widthwise inner edges 41A) in the width direction are interposed between the absorber and the top sheet in the thickness direction; the pair of side sheets extend outwardly from the top sheet and the absorber in the width direction; and the side sheets have a higher density than the top sheet.

### Brief Description of Drawings

Fig. 1 is a plan view of an absorbent article according to a first embodiment, seen from its skin-contacting surface side.
Fig. 2 is a schematic cross-sectional view taken along the A-A cross-section shown in Fig. 1.

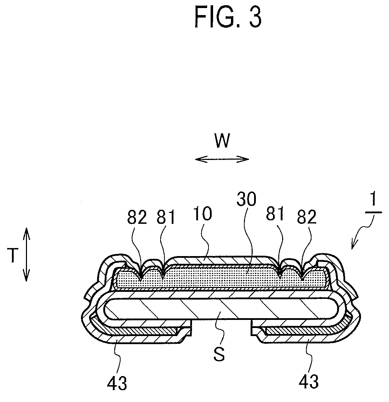

Fig. 3 is a schematic view illustrating a state in which the absorbent article shown in Fig. 1 is worn.
Fig. 4 is a schematic cross-sectional view of an absorbent article according to a modification example.

### Description of Embodiments

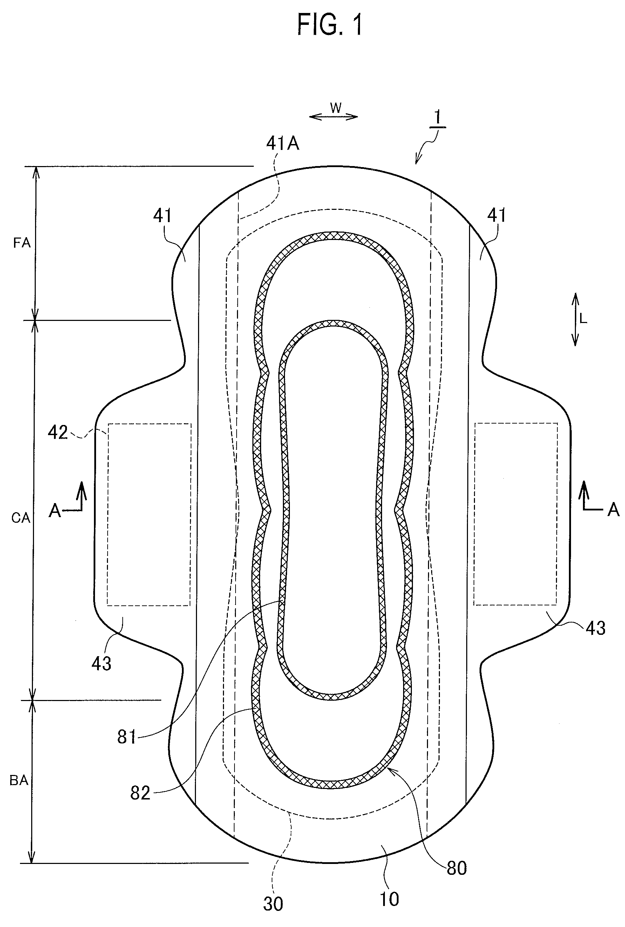

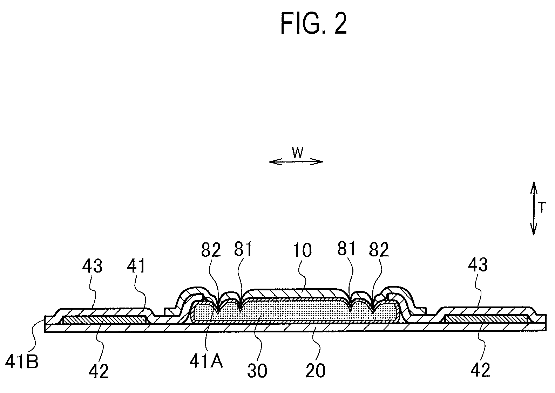

With reference to the drawings, an absorbent article 1 according to an embodiment will now be explained. Fig. 1 is a plan view of the absorbent article; and Fig. 2 is a cross-sectional view taken along the A-A shown in Fig. 1. The absorbent article 1 according to the embodiment is a sanitary napkin, for example.

An absorbent article according to the embodiment has: a longitudinal direction extending between the wearer front side and the wearer back side; a width direction orthogonal to the longitudinal direction; and a thickness direction having an inward direction toward the wearer and an outward direction opposite to the inward direction. The absorbent article is a day-time sanitary napkin. Thus, in the absorbent article according to the embodiment, a front region FA on the wearer front side and a back region BA on the wearer back side have substantially the same lengths in the longitudinal direction.

It is noted that, a region between a pair of wings (to be described later) is a central region CA including a vaginal area-contacting region to be disposed adjacent the vaginal area of the wearer. The front region FA is in the front relative to the central region CA and the back region BA is in the back relative to the central region CA.

It is noted that, although the embodiment uses a day-time sanitary napkin by way of example, the absorbent article of the invention may also be applied to a night-time sanitary napkin. Such a night-time sanitary napkin has a configuration in which the length of the back region BA on the wearer back side is longer than that of the front region FA on the wearer front side, in the longitudinal direction.

The absorbent article 1 has a top sheet 10 to contact the wearer's skin, a liquid-impermeable back sheet 20 that is impermeable to fluid, an absorber 30, and a pair of side sheets 41. The absorber 30 is interposed between the top sheet 10 and the back sheet 20. Thus, the absorber 30 is shown in dotted lines in Fig. 1. The absorber 30 is disposed in the center in the longitudinal direction L and the width direction W of the absorbent article 1. The absorbent article 1, in the plan view of Fig. 1, has wings 43 disposed outside of the absorber 30 in the width direction W that is orthogonal to the longitudinal direction L.

The top sheet 10 is a liquid-permeable sheet that is permeable to bodily fluid such as menses. The top sheet 10 covers at least a top surface of the absorber 30 and widthwise inner edges of the side sheets 41. There is no particular limitation for the top sheet 10 as long as it is a sheet material having a liquid-permeable structure, for example, a nonwoven fabric, a woven fabric, a porous plastic sheet, and a mesh sheet. Both natural and chemical fibers can be used as materials for woven and nonwoven fabrics. The top sheet of the embodiment is an air-through nonwoven fabric having a basis weight of 23 g/m² and a density of 0. 045 g/m³.

The basis weight and density of material can be determined through the following approach, for example. The absorbent article is taken out of a pouch if it is contained therein and is unfolded and flattened. A portion of interest, whose basis weight and density are to be determined, is designated and the thickness and the area of the designated portion are measured. Next, the designated portion, whose basis weight and density are to be determined, is cut from the absorbent article and weighed. Other components than the material of interest are then removed from the cut portion, and the weight of the material of interest is measured. The basis weight is calculated based on the weight of the material of interest and the area of the designated portion, whose basis weight and density are to be determined. Finally, the density is calculated based on the basis weight and the thickness.

Examples of natural fibers include cellulose such as comminuted pulp and cotton. Examples of chemical fibers include: regenerated cellulose, such as rayon and fibrillated rayon; semi-synthetic cellulose, such as acetate and triacetate; and hydrophobic thermoplastic chemical fibers or hydrophilized hydrophobic thermoplastic chemical fibers.

Examples of hydrophobic thermoplastic chemical fibers include: mono-filaments made of polyethylene (PE), polypropylene (PP), polyethylene terephthalate (PET), or the like; fibers made of grafted PE and PP; and composite fibers having, for example, a core-sheath structure.

In particular, for nonwoven fabrics, any dry process (e.g., carding, spunbonding, melt blown, and air laid processes), wet process, and a combination thereof may be used in web forming. Further, a bonding process may include, by way of example, but without limitation, thermal bonding, needle punching, and chemical bonding. A spunlace sheet formed by hydroentanglement may also be used.

For a porous plastic sheet, a porous sheet made of thermoplastic resin such as PE, PP, or PET, and a porous foam material may be used, for example. To use such materials, preferably a filler formed of, for example, titanium oxide or calcium carbonate may be mixed by a percentage of 0.5 to 10% so as to provide opacity, as needed. Further, a film made of thermoplastic resin, as mentioned above, may be perforated, heat-embossed, or machine-processed to create a porous film for use. A porous film may be used in a laminate sheet, combined with a nonwoven fabric.

It is noted that the top sheet 10 may overlap at least a portion of each side sheet 41, more preferably, the top sheet 10 overlaps at least one-third of the entire widthwise length of the side sheet 41.

The back sheet 20 and the top sheet 10 have substantially the same lengths. The back sheet 20 is formed of a material(s) different from the top sheet. A laminate nonwoven fabric formed of, for example, a polyethylene sheet and polypropylene as the majority components; a breathable resin film; or a sheet prepared by joining a spunbond or spunlace nonwoven fabric with a breathable resin film, may be used. The back sheet 20 is preferably formed of a material that can provide some flexibility to an extent that a wearer does not feel uncomfortable while wearing the absorbent article; for example, a film whose majority component is a low density polyethylene (LDPE) resin, having a basis weight of 15-30 g/m², may be used. The back sheet 20 is preferably impermeable to liquid yet permeable to moisture; for example, it can be formed by melting an inorganic filler and kneading it with an olefinic resin such as polyethylene or polypropylene, and then stretching the mixture into a microporous sheet.

The absorber 30 contains hydrophilic fibers and pulp. The absorber 30 is formed of a material(s) that can absorb bodily fluid such as menses. For example, cellulose, such as comminuted pulp and cotton; regenerated cellulose, such as rayon and fibrillated rayon; semi-synthetic cellulose, such as acetate and triacetate; particulate polymer; fibrous polymer; hydrophobic thermoplastic chemical fibers or hydrophilized hydrophobic thermoplastic chemical fibers; and air laid pulp treated with chemical bonding, for example, may be used alone or in mixture.

Non-limiting examples of process for forming these materials into an absorber can include air laid, melt blown, spunlacing, and papermaking processes, through which the materials are formed into an absorbent sheet. Cellulose foam and continuous resin foam, for example, may also be used for absorber. Further, foams or the sheeted materials, as mentioned above, may be comminuted and then formed into an absorber for use. Among the absorber examples described above is an absorber that has been formed by mixing pulp and particulate polymer by a percentage of 80-100% and 20-0%, respectively, wrapping the mixture with tissue paper, and then embossing and sheeting, which sheet having a basis weight of 100-2000 g/m² and a caliper of 1-50 mm. Here, the embossing is for keeping the shape of the absorber, and the percentage of the embossed area is 10-100%, preferably 30-80%.

The absorber 30 may be formed by air laid process in which hydrophilic fibers or powders are laid above one another; or the absorber may be an air-laid sheet made through air laid process in which hydrophilic fibers or powders are formed into a sheet; or the absorber may be formed by distributing comminuted pulp mixed with superabsorbent polymer on tissue paper (for example, tissue paper having a basis weight of 15 g/m²) and then wrapping up the pulp and polymer with the tissue paper.

Further, other examples of material for a thin absorber can include absorbent sheets and polymer sheets, preferably having a thickness of 0.3-5 mm. There is no limitation for such absorbent sheets and polymer sheets as long as they are typically used for absorbent articles such as sanitary napkins.

Examples of absorbent sheets include pulp sheets formed by adding binder to absorbent paper, nonwoven fabrics, and fibers; and polymer sheets can include sheet materials made by mixing comminuted pulp or fibers with particulate polymer and sheeting the mixture. It is noted that, such sheets made by mixing fibers and particulate polymer may have the particulate polymer dispersed in layers or three-dimensionally therein, and either type is usable.

Preferable fibers used in making absorbent sheets and polymer sheets can include: cellulose fibers, such as wood pulp; regenerated cellulose fibers, such as rayon and cuprammonium rayon; hydrophilic synthetic fibers such as polyvinyl alcohol fibers and polyacrylonitrile fibers; or polyethylene, polypropylene, polyethylene terephthalate, polyethylene/polypropylene composite fibers, polyethylene/polyethylene terephthalate composite fibers, whose fiber surfaces have been hydrophilized, treated with surfactant or the like, but cellulose fibers are more preferable because the good affinity for water is ensured.

Preferable particulate polymer used for polymer sheets is one that can absorb and retain a liquid at least 20 times its weight and turn the liquid into gel, and examples can include starch, cross-linked carboxymethyl cellulose, polyacrylic acid and salts thereof, and graft polymer of polyacrylic acid salt, and the like.

The absorber 30 according to the embodiment is formed of pulp, for example, cotton-like pulp or synthetic pulp, that is layered to have a basis weight of about 100-300 g/m² and wrapped with protective paper (not shown). The protective paper keeps the shape of pulp; and crepe paper or tissue paper is usable as the protective paper, for example.

The absorber 30 has an elongate shape in the longitudinal direction, and is smaller than the back sheet 20 in size. The length in the width direction W of the absorber 30 is about 50-80 mm, generally corresponding to the sideway dimension of adult female's crotch area. The absorber 30 is fixed to the back sheet 20 via adhesive such as hot melt adhesive.

The side sheets 41 cover the widthwise side edges of the absorber 30. The side sheets 41 are formed of a material(s) different from the top sheet 10 and the back sheet 20. There is no particular limitation for the side sheet 41 material as long as it is a sheet material having a liquid-permeable structure, for example, a nonwoven fabric, a woven fabric, a porous film, and a mesh sheet. Examples of nonwoven fabric forming the side sheets 41 can include spunbond nonwoven fabrics, spunlace nonwoven fabrics, SMS nonwoven fabrics, point-bonded nonwoven fabrics, and air-through nonwoven fabrics. From the viewpoint of securing the strength of the side sheets 41, the side sheets 41 are preferably formed of a SMS nonwoven fabric. More detailed explanation of the side sheets 41 and the top sheet 10 will be provided in the following.

The widthwise inner edges 41A of the side sheets 41 are interposed between the absorber 30 and the top sheet 10 in the thickness direction. That is, in a region where the top sheet 10, the side sheet 41, the absorber 30, and the back sheet 20 are layered, they are disposed in order in which they are described, starting from the wearer skin-contacting surface side, moving down. Lateral outer edges 41 B of the side sheets 41 are on the skin-contacting surface side of the back sheet 20 and aligned with the widthwise side edges of the back sheet 20. The side sheets 41 cover part of side margins of the absorber 30 and wings 43.

It is noted that the side sheets 41 may cover at least a portion of the absorber 30 and the top sheet 10 and are not limited to the configuration of the embodiment. Specifically, the widthwise outer edges of the side sheets 41 may be disposed inside of the widthwise side edges of the back sheet 20 in the width direction.

In the absorbent article 1, the peripheries of the top sheet 10, the side sheets 41, and the back sheet 20 are joined, encasing the absorber 30 therein. To join the top sheet 10 and the back sheet 20, any one of heat embossing, ultrasonic, and hot melt adhesive, or a combination thereof may be employed.

Adhesive (not shown) is applied in several regions on a surface of the back sheet 20 that is configured to contact an underwear (S). The adhesive is intermittently disposed along the longitudinal direction L on a back side of the absorber 30. An underwear-contacting surface of each of the wings 43 is also provided with adhesive. The adhesive is in contact with a releasable sheet (not shown) before the time of use. The releasable sheet protects the adhesive from deteriorating until the time of use. At the time of use, the releasable sheet is removed by the wearer.

It is noted that, in an absorbent article that does not employ such releasable sheet, a wrapper sheet that individually wraps the absorbent article may be adapted to protect the adhesive from deteriorating until the time of use. When the adhesive and the wrapper sheet are in contact with each other, a surface of the wrapper sheet preferably has been treated so that it is releasably affixed to the adhesive without deteriorating the adhesion of the adhesive.

A compression portion 80 is formed in the absorbent article 1 by compressing the top sheet 10 and the absorber 30 in the thickness direction. To form the compression portion 80, the skin-contacting side of the top sheet and the non-skin-contacting side (i.e., garment-contacting surface side) of the absorber 30 of the absorbent article 1 are processed to form a groove. The compression portion 80 includes: a ring-shaped first compression portion 81 and a ring-shaped second compression portion 82 disposed outside of the first compression portion in the width direction and outside of the first compression portion in the longitudinal direction.

Further, the side sheets 41 have a higher density than the top sheet 10. When the top sheet 10 and the side sheets 41 are formed of nonwoven fabrics, the side sheets 41 have a higher filament density than the top sheet 10. The density of the side sheets 41 may be adapted so as to be higher than the density of the top sheet 10, and thus, the side sheets may have a lower filament basis weight than the top sheet 10.

An exemplifying combination of a top sheet and a side sheet can be one having a top sheet formed of an air laid nonwoven fabric, point-bonded nonwoven fabric, or air-through nonwoven fabric, having a density of 0.045-0.098, and a side sheet formed of a spunbond nonwoven fabric or SMS nonwoven fabric, having a density of at least 0.1.

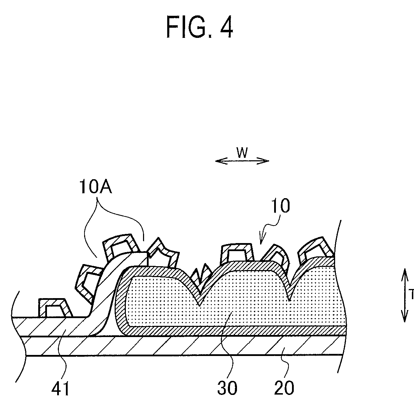

When the top sheet 10 is formed of a porous film having a plurality of pores 10A passing therethrough in a thickness direction T, voids created by the pores lower the relative density of the top sheet 10. Fig. 4 shows a schematic cross-sectional view illustrating an embodiment in which a top sheet 10 is formed of a porous film. The schematic cross-sectional view shows a cross section that is orthogonal to the longitudinal direction and parallel to the width direction of the absorbent article.

An exemplifying combination of a top sheet formed of such porous film and a side sheet can be one having a top sheet formed of a porous film, having a density of 0.05-0.5, and a side sheet formed of a nonwoven fabric or non-breathable film, having a density of at least 0.1. Further, a porous film forming the top sheet preferably has a porosity of 1-9%, more preferably 20-80%, and more preferably 10-30%.

Here, "porosity" is a ratio (%) of the pore area to the total area. The porosity can be determined using the following approach, for example. A 50 mm X 50 mm sample is prepared, and then the area of a single pore in the sample is measured using a microscope. By multiplying the measured area of the single pore by the number of the pores in the sample, the pore area of the sample is obtained. By dividing the obtained pore area by the area of the sample, the porosity is calculated.

Menses remaining on the widthwise side edges of the absorber 30 and on the widthwise side margins of the absorber 30 will stay on the top sheet 10. Here, due to the relatively high density of the side sheets 41 and the relatively low density of the top sheet 10, menses will be drawn from the top sheet 10 having lower density to the side sheets 41 having higher density. Thus, menses passed into region which is outside of the absorber in the width direction are prevented from staying on the top sheet 10.

When the absorbent article 1 is used over an extended period of time, for example, menses can soil the absorbent article 1 in its entirety, so that a wearer may find it difficult to decide from its appearance whether or not she should change the absorbent article 1. Further, when the absorbent article 1 is used over an extended period of time, the absorber 30 can be contracted in the width direction, causing menses to pass into the wings 43. In particular, menses can be locked in the side sheets 41, the sight of which can concern the user about leakage. Furthermore, the soiled side sheets 41 can contaminate the user's clothing.

Such concerns about leakage and soiling felt by users, however, can be eased by reducing the amount of menses staying on the top sheet 10, by covering the widthwise inner edges of the side sheets 41 with the top sheet 10 so that the menses on the top sheet 10 are smoothly transported to the absorber 30 via the side sheets 41. According to the absorbent article of the embodiment, even during an extended period of use, leakage in the crotch region of the absorbent article is prevented, and wearer's concerns about leakage during use can be eased.

Further, due to the density structures of the side sheets and the top sheet, menses that have passed into wings 43, if any, can be drawn to the non-skin-contacting surface side, so that soiling on the skin-contacting side can be reduced and that a user can use the product anxiety-free without concerning about leakage and discomfort during use.

The portions in which the top sheet 10, the absorber 30, and the side sheets 41 overlap each other are joined via adhesive alone but not via thermal bonding or ultrasonic bonding. If the components are bonded via thermal bonding, for example, a high density area would be partially created due to the bonding. This can invalidate the density structures of the side sheets 41 and the top sheet 10, canceling the drawability to the side sheets 41. Further, such high density area that is created partially can block the folding of the wings at an intended location when the absorbent article is being worn. By simply bonding via adhesive the portions in which the top sheet 10, the absorber 30, and the side sheets 41 overlap each other, creation of high density area is prevented, so that the inconvenience can be avoided. In this way, any base or heat source otherwise required in thermal bonding and ultrasonic bonding is unnecessary, enabling a simple manufacturing equipment.

Further, if the components are bonded via thermal bonding, for example, the portions treated with the thermal bonding can be recessed, thereby decreasing the volume of inter-fiber voids, or the portions can be melted, so that the portions can lose drawability of bodily fluid. By simply bonding via adhesive, drawability loss can be prevented.

For such adhesive, a rubber-based or polyolefin-based adhesive is preferable. Alternatively, from the viewpoint of adhesive strength, an olefin-based, hot-melt type adhesive is preferable. Exemplifying adhesive coating methods can include: slot coating for dispensing adhesive through slots, omega coating for producing omega-pattern adhesive coating, spiral coating for producing spiral-pattern adhesive coating, and bead coating for linear adhesive coating. Direct coating of adhesive onto a substrate using a coater is more preferable.

Joint sections of the side sheets 41 and the top sheet 10 are provided in regions in which the top sheet 10, the side sheets 41, and the absorber 30 overlap each other. Here, the regions in which the top sheet 10, the side sheets 41, and the absorber overlap each other are the regions in which the top sheet 10, the side sheets 41, and the absorber are layered in the thickness direction, and include not only the regions in which the components directly overlap each other but also the regions in which the components indirectly overlap each other. The joint sections of the side sheets 41 and the top sheet 10 are disposed in at least part of the regions in which the top sheet 10, the side sheets 41, and the absorber 30 overlap each other. According to this configuration, the components are in close contact with each other, whereby menses on the top sheet 10 are more readily drawn to the side sheets 41, which menses can be absorbed in the absorber 30 by way of the side sheets 41.

Further, the side sheets 41 and the back sheet 20 are bonded via adhesive, for example, and united. This strengthens the wings 43 against tear. Furthermore, because the side sheets 41 are united with the back sheet 20 that is liquid-impermeable, the wings 43 will more reliably prevent leakage. It is noted that, although reinforcement sheets 42 are interposed between the side sheets 41 and the back sheet 20 in the individual wings 43 in the embodiment, this configuration is not a limitation. In an absorbent article 1 according to a modification example, a reinforcement sheet may be omitted, or a reinforcement sheet may extend to the widthwise outer edge of a side sheet.

The content of the present invention has been disclosed as above through the embodiments of the present invention; however, the description and drawings as part of the disclosure should not be understood as limitation to the invention. After reviewing this disclosure, various alternative embodiments, examples, and practicable techniques will be apparent to those skilled in the art.

In a modification example, a compression portion 80 may be formed in regions in which a top sheet 10, side sheets 41, and an absorber 30 overlap each other. According to this configuration, menses on the top sheet 10 can be efficiently drawn to the side sheets 41 and the absorber 30 by way of the compression portion.

It is appreciated that Japanese Patent Application No. 2013-204166 (filed on September 30, 2013) is incorporated herein in its entirety by reference.

### Industrial Applicability

An absorbent article, which can prevent leakage of menstrual flow in the crotch region of the absorbent article even during long hours of use, thereby easing wearer concerns about leakage during use, can be provided.

### Reference Signs List

1: absorbent article
10: top sheet
10A: pore
20: back sheet
30: absorber
41: side sheet
41A: widthwise inner edge
41B: widthwise outer edge
42: reinforcement sheet
43: wing
80: compression portion
81: first compression portion
82: second compression portion
BA: back region
CA: central region
FA: front region
L: longitudinal direction
W: width direction

## Claims

1. An absorbent article comprising:
a longitudinal direction;
a width direction orthogonal to the longitudinal direction;
a thickness direction;
a liquid-permeable top sheet;
a liquid-impermeable back sheet;
an absorber interposed between the top sheet and the back sheet; and
a pair of side sheets covering widthwise side edges of the absorber, wherein
widthwise inner edges of the pair of side sheets are interposed between the absorber and the top sheet in the thickness direction,
the pair of side sheets extend outwardly from the top sheet and the absorber in the width direction, and
the side sheets have a higher density than the top sheet.

2. The absorbent article according to claim 1, wherein the top sheet and the side sheets are formed of nonwoven fabric.

3. The absorbent article according to claim 1, wherein
the top sheet is formed of a porous film having a plurality of pores which pass through the top sheet in the thickness direction; and
the side sheets are formed of a nonwoven fabric.

4. The absorbent article according to any one of claims 1 to 3, wherein portions in which the top sheet, the absorber and the side sheets overlap each other are bonded via only adhesive.

5. The absorbent article according to any one of claims 1 to 4, wherein joint sections in which the side sheets and the top sheet are joined each other are disposed in regions in which the top sheet, the side sheets and the absorber overlap each other.

6. The absorbent article according to any one of claims 1 to 5, wherein a compression portion that is formed by compressing the side sheets, the top sheet, and the absorber in the thickness direction is formed in regions in which the top sheet, the side sheets and the absorber overlap each other.
